# EUROPEAN PATENT SPECIFICATION

(11) **EP 3 680 013 B1**
(45) Date of publication and mention of the grant of the patent: **29.11.2023**
(21) Application number: 18853595.9
(22) Date of filing: 31.08.2018
(51) Int. Cl.: B01J 20/12, B01D 15/00, B01J 20/28, B01J 20/30

(54) **ADSORBENT MATERIAL FOR PURINE BASE, ADSORPTION FILTER FOR PURINE BASE USING SAME, ADSORPTION COLUMN FILLER FOR PURINE BASE, AND SYSTEM FOR REMOVING PURINE BASE USING THESE**
ADSORPTIONSMATERIAL FÜR PURINBASE, ADSORPTIONSFILTER FÜR PURINBASE DAMIT, ADSORPTIONSSÄULENFÜLLSTOFF FÜR PURINBASE UND SYSTEM ZUR ENTFERNUNG VON PURINBASE DAMIT
MATÉRIAU ADSORBANT POUR BASE PURIQUE, FILTRE D'ADSORPTION POUR BASE PURIQUE L'UTILISANT, CHARGE DE COLONNE D'ADSORPTION POUR BASE PURIQUE, ET SYSTÈME D'ÉLIMINATION DE BASE PURIQUE LES UTILISANT

(30) Priority: 05.09.2017 JP 2017169922
(43) Date of publication of application: 15.07.2020
(73) Proprietor: National Institute for Materials Science, Tsukuba-shi, Ibaraki 305-0047 (JP)
(72) Inventor: TAMURA Kenji, Tsukuba-shi Ibaraki 305-0047 (JP); SAKUMA Hiroshi, Tsukuba-shi Ibaraki 305-0047 (JP)
(74) Representative: Vossius & Partner Patentanwälte Rechtsanwälte mbB
(86) International application number: PCT/JP2018/032352
(87) International publication number: WO 2019/049782

(56) References cited:
- JP-A- S6 297 641
- JP-A- H05 139 720
- JP-A- H06 142 405
- JP-A- 2004 222 719
- JP-A- 2012 125 205
- JP-A- 2015 171 670
- JP-A- 2017 001 030
- JP-A- 2017 136 584
- US-A- 5 360 775
- US-A- 5 614 096

## Description

### Field of the Invention

The present invention relates to a technique capable of adsorbing and separating purine bases from an aqueous solution and, more particularly, to a purine base adsorption material using a layered clay mineral, a purine base adsorption filter using the same, a purine base adsorption column filler, and a purine base removal system using them.

### Background Art

Caffeine that is a sort of alkaloid is contained in large amounts in a variety of plants inclusive of a plant providing a raw material for favorite beverages such as coffee or tea. As caffeine has strong central nervous excitability, its excessive intake causes even healthy persons to suffer from symptoms such as extreme excitement, over-sensitiveness, nausea and insomnia. This, combined with recent health inclinations, results in a demand for development of a technology capable of effective reduction of caffeine from beverages. With such background in mind, numerous techniques of using active carbon, acid clay or activated clay as adsorbents have been studied with a view to caffeine removal.

In particular, how to reduce caffeine from in an aqueous solution such as tea extracts has been under study. For instance, a prior art (see Patent Publication 1 as an example) discloses a technique of bringing activated clay or acid clay in contact with a caffeine-containing aqueous solution thereby removing caffeine from the aqueous solution while care is taken of reductions in catechin. Another prior art (see Patent Publications 2 or 3 as an example) discloses a process of producing refined green tea extracts improved in terms of flavor by way of a step of mixing a tea extract with an ethanol aqueous solution and bringing the mixture in contact with at least one selected from the group of active carbon, acid clay or activated clay and a step of processing the resultant product with tannase. Many other techniques have been disclosed so far. For instance, a process of bringing an aqueous liquid containing caffeine and oxalic acid in contact with a processing agent comprising acid clay and/or activated clay thereby removing them is disclosed (see Patent Publication 4), and a process of producing refined tea extracts wherein the content of caffeine in a tea extract is reduced by acid clay in contact with cations without deterioration of its appearance and flavor is disclosed (see Patent Publication 5).

Further, a filtration material has been proposed for use for removal of a specific component including caffeine from the fluid to be filtrated. For instance, a filter comprising a cellulosic fiber as well as a polyamine-epichlorohydrin resin, acid clay and activated clay is disclosed (see Patent Publication 6 as an example).

Furthermore, it has been known that the aforesaid acid clay or activated clay is also capable of adsoprtion of purine bases other than caffeine, for instance guanosine and guanine (see Patent Publication 7 or 8 as an example). The content of purine bases contained in alcoholic beverages like beer is also required to be much lower as is the case with caffeine, because they cause diseases such as gout.

Patent Publication 9 discloses a compressed adsorbent filter cake for removing caffeine from liquids. Patent Publication 10 discloses a porous clay intercalation compound which is suitable for use in e.g. catalysts, absorption separating agents, or deodorizers.

Patent Publication 11 discloses a method for producing an inorganic layered porous body used for e.g. an adsorbent, a catalyst, or a carrier.

### Prior Arts

### Patent Publications

Patent Publication 1: JP(A) 6-142405
Patent Publication 2: JP(A) 2004-222719
Patent Publication 3: JP(A) 2007-89561
Patent Publication 4: JP(A) 2011-19469
Patent Publication 5: JP(A) 2014-212743
Patent Publication 6: JP(A) 2015-171670
Patent Publication 7: JP(A) 2017-1030
Patent Publication 8: JP(A) 2017-136584
Patent Publication 9: US 5,614,096
Patent Publication 10: US 5 360 775
Patent Publication 11: JP H05 1 39720 A

### Summary of the Invention

### Problems with the Prior Art

However, one of the most significant problem with the prior art using acid clay or activated clay as the adsorption material for purine bases inclusive of caffeine, guanosine and guanine is that even under the optimized adsorption conditions, the purine bases remain in some considerable amounts because of insufficient selectivity of these adsorption materials for purine bases. To achieve sufficient removal of purine bases, therefore, it is necessary to take some action such as an increased number of processing steps, resulting in adverse influences on production cost.

With the aforesaid prior art in mind, the present invention has for its object to provide a purine base adsorption material capable of efficient reduction of purine bases, a purine base adsorption filter using the same, a purine base adsorption column filler, and a purine base removal system using them.

### Embodiments of the Invention

For the purpose of achieving the aforesaid object, the purine base adsorption material according to the present invention contains a 2:1 type layered clay mineral as represented by the following general formula:

[(E1^{m+}_{a/m}E2⁺_{b})(M1_{c}M2_{ct})(Si₄₋ₑAlₑ)O₁₀(OH_{f}F_{2-f})]

where
m is a natural number of 2 to 4,
parameters m, a, b, c, d, e and f satisfy inequalities: 0.2≦a+b<0.75, 0.12≦a≦0.6, 0≦b, 0≦c≦3.0, 0≦d≦2, 2≦c+d≦3, 0≦e<4, and 0≦f≦2,
E1 is at least one element selected from the group consisting of Mg, Al, Si, Sc, Ca, Sr, Ti, V, Cr, Mn, Fe, Co, Ni, Cu, Zn, Ga, Zr and Ba, said element turning into a polyvalent cation between layers,
E2 is at least one element selected from the group consisting of Na, Li and K, said element turning into a monovalent cation between layers,
M1 is at least one metal element selected from the group consisting of Mg, Fe, Mn, Ni, Zn and Li, and
M2 is at least one metal element selected from the group consisting of Al, Fe, Mn and Cr, wherein:
   said M1 and M2 form an octahedral sheet.

According to one embodiment of the invention, the 2:1 type layered clay mineral may be at least one smectite selected from the group consisting of montmorillonite, beidellite, nontronite, saponite, hectorite and stevensite.

According to one embodiment of the invention, the parameters a and b may satisfy 0.2≦a+b<0.6.

According to one embodiment of the invention, the E1 may be at least one element selected from the group consisting of Mg, Al, Si, Sc, Ca, Ti, V, Cr, Mn, Fe, Ni, Cu, Ga and Zr.

According to one embodiment of the invention, an occupied area per polyvalent cation E1^{m+} on a layer plane may be in a range of no less than 0.8 nm²/cation to no greater than 9 nm²/cation.

According to one embodiment of the invention, the 2:1 type layered clay mineral may have lattice spacing of a basal plane of no less than 1.2 nm to no greater than 1.9 nm as measured by X-ray diffraction in a state wetted with water.

According to one embodiment of the invention, the 2:1 type layered clay mineral may have a primary particle diameter (viz., a constant direction (Green) diameter in the ab-axis direction of a crystal observed under a microscope) of no less than 10 nm to no greater than 2 µm.

According to one embodiment of the invention, the E1 may be at least one element selected from the group consisting of Al, Si, Sc, Ti, V, Mn, Fe, Ni, Cu and Ga.

According to one embodiment of the invention, the E1 may be at least one selected from the group consisting of Al, Si, Cr, Fe, Ga and Zr.

According to one embodiment of the invention, the purine base adsorption material may further contain at least one additive selected from the group consisting of active carbon, acid clay, activated clay and zeolite.

For the purpose of achieving the aforesaid object, the purine base adsorption filter comprises the purine base adsorption material carried on a fibrous material.

According to one embodiment of the invention, the fibrous material may comprise a thermoplastic polymer selected from the group consisting of a polyolefin, a polyamide and a polyester.

For the purpose of achieving the aforesaid object, the purine base adsorption column filler of the invention includes the aforesaid purine base adsorption material that is spherically granulated.

According to one embodiment of the invention, the granulated material may have a mean particle diameter of no less than 1 µm to no greater than 5 mm.

For the purpose of achieving the aforesaid object, the purine base removal system of the invention comprises a feeding means for feeding a fluid containing purine bases, a removal means for removing purine bases from the fluid fed from the feeding means, and a recovery means for recovering the fluid from which the purine bases are removed by the removal means, wherein the removal means comprises the purine base adsorption filter or the purine base adsorption column filler.

### Advantages of the Invention

The purine base adsorption material of the invention contains a 2:1 type layered clay mineral represented by the aforesaid general formula, and has a given polyvalent cation positioned between layers, making it possible to efficiently and selectively adsorb and separate purine bases from an aqueous solution containing purine bases at low cost. The selectivity for purine bases is much more improved in a low concentration region in particular, and swelling in an aqueous solution is held back, contributing more to simplification of the separation steps and curtailment of filtration time. If such a purine base adsorption material is used, it is then possible to provide a purine base adsorption filter capable of selective adsorption and removal of purine bases, a purine base adsorption column filler, and a purine base removal system using them.

### Brief Explanation of the Accompanying Drawings

Fig. 1 is a set of schematic views illustrative of an existing 2:1 type layered clay mineral, and a 2:1 type layered clay mineral and its derivative that form a purine base adsorption material according to one embodiment of the invention.
Fig. 2 is a schematic view of a purine base removal system according to one embodiment of the invention.
Fig. 3 is a diagram for the UV-vis spectra of a supernatant of an Example 1 sample after adsorption processing.
Fig. 4 is a diagram for the UV-vis spectra of supernatants of samples of Example 12 and Comparative Example 11 after adsorption processing.

### Modes for Carrying Out the Invention

Some inventive modes of the invention will now be explained with reference to the drawings. It is here understood that like elements are provided with like reference numerals; so they will not be explained anymore. It is also noted that the words "a" and "one", as used in the claims and in the corresponding portion of the specification, are defined as including one or more of the referenced ions or elements unless otherwise stated.

### Mode 1 of the Invention

Referring to Mode 1, the purine base adsorption material and how to produce it according to one embodiment of the invention are explained. While, in each of the following modes of the invention, the adsorption of caffeine typical of purine bases, viz., a caffeine adsorption material is explained, it is understood that the inventive purine base adsorption material may apply equally to other purine bases such as purine, adenine, guanine, hypoxanthine, xanthine, theobromine, uric acid and isoguanine.

Focusing on a 2:1 type layered clay mineral having a layer charge of no less than 0.2 to less than 0.75 as described later, the inventors have discovered that if a polyvalent cation is introduced between its layers, caffeine adsorption capability much higher than that of acid clay or activated clay used heretofore in the art is then obtained, arriving at the invention of a caffeine adsorption material and its use, as mentioned below in details.

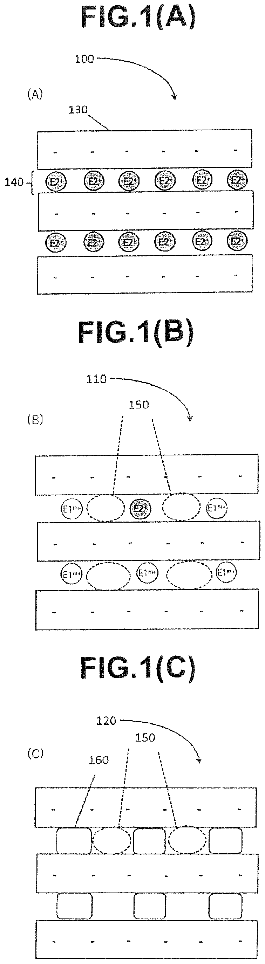

Fig. 1 is a set of schematic views illustrative of an existing 2:1 type layered clay mineral, and a 2:1 type layered clay mineral and its derivative that form a purine base adsorption material according to one embodiment of the invention.

More specifically, Fig. 1(A) is a schematic view of an existing 2:1 type layered clay mineral 100; Fig. 1(B) is a schematic view of a 2:1 type layered clay mineral 110 according to one embodiment of the invention; and Fig. 1(C) is a schematic view of a derivative of the inventive 2:1 type layered clay mineral 120. A layered clay mineral is here briefly explained. The layered clay mineral is finely classified depending on its constitutional elements and layer charges, and its fundamental crystal structure comprises a tetrahedral sheet in which tetrahedrons, each having four O²⁻ coordinated at a metal, mainly silicon or aluminum, are joined together in a hexagonal mesh shape and an octahedral sheet consisting of edge-sharing octahedrons having six H⁻ or O²⁻ coordinated at trivalent, divalent or monovalent metals such as aluminum, magnesium or lithium. This tetrahedral sheet is joined to the octahedral sheet with the sharing of apex oxygen: a layer comprising one octahedral sheet joined to one tetrahedral sheet is called a 1:1 layer, and a layer comprising two tetrahedral sheets joined to both sides of one octahedral sheet is called a 2:1 layer (130 in Fig. 1). In the present disclosure, a clay mineral comprising this 2:1 layer will be called a 2:1 type layered clay mineral 100 (Fig. 1(A)).

Referring to this 2:1 type layered clay mineral 100, when there is short of positive charges due to isomorphous replacement in its structure, cations 140 corresponding to the amount of replacement will exist as exchangeable cations between the layers.

The then negative charge of the 2:1 type layer is called the layer charge, and the 2:1 type layered mineral is broken down by this value (indicated by the absolute value of charges per 2:1 type composition formula). For instance, 0 for talc and pyrophyllite, 0.2 to 0.6 for smectites, 0.6 to 0.9 for vermiculite, 0.6 to 1.0 for mica and mica clay minerals, 0.8 to 1.2 for chlorite, and ~2 for brittle mica.

The 2:1 type layered clay mineral represented by the following composition formula (Fig. 1(B)) is used as the caffeine adsorption material of the invention.

[(E1^{m+}_{a/m}E2⁺_{b})(M1_{c}M2_{d})(Si₄₋ₑAlₑ)O₁₀(OH_{f}F_{2-f})]

where
m is a natural number of 2 to 4,
parameters m, a, b, c, d, e and f satisfy inequalities: 0.2≦a+b<0.75, 0.12≦a≦0.6,
0≦b, 0≦c≦3.0, 0≦d≦2,
2≦c+d≦3, 0≦e<4, and 0≦f≦2,
E1 is at least one element selected from the group consisting of Mg, Al, Si, Sc, Ca, Sr, Ti, V, Cr, Mn, Fe, Co, Ni, Cu, Zn, Ga, Zr and Ba, the element turning into a polyvalent cation between layers,
E2 is at least one element selected from the group consisting of Na, Li and K, the element turning into a monovalent cation between layers,
M1 is at least one metal element selected from the group consisting of Mg, Fe, Mn, Ni, Zn and Li, and
M2 is at least one metal element selected from the group consisting of Al, Fe, Mn and Cr, wherein:
   the M1 and M2 form the aforesaid octahedral sheet.

Referring to Fig. 1(A) and Fig. 1(B) showing the 2:1 type layered clay mineral 110 forming the caffeine adsorption material of the invention (hereinafter called the inventive 2:1 type layered clay mineral for short), while a cation 140 positioned between the layers may contain E2⁺ that is a monovalent cation, it is understood that the cation 140 is characterized by containing at least E1^{m+} that is a polyvalent cation.

In the existing 2:1 type layered clay mineral 100 shown in Fig. 1(A), a 2:1 layer 130 has a charge of -6, and six or a total of twelve monovalent cations are positioned between the adjacent layers in such a way as to compensate for such a negative charge. In the inventive 2:1 type layered clay mineral shown in Fig. 1(B), on the other hand, 11 out of 12 monovalent cations of the existing 2:1 type layered clay mineral 100 are ion exchanged with, for instance, four polyvalent (divalent) cations and one polyvalent (trivalent) cation. Although Fig. 1 shows that the 2:1 layer 130 has a charge of -6 and a portion of monovalent cations for compensating for that charge is replaced or substituted by polyvalent cations, it is here noted that the present invention is not limited thereto. As a matter of course, all monovalent cations may be replaced by polyvalent cations.

The inventive 2:1 type layered clay mineral 110 is represented by the aforesaid composition formula. If the parameters m, a and b satisfy the aforesaid ranges, the layer charge then comes within the range of no less than 0.2 to less than 0.75, resulting in enhanced caffeine absorption capability. If the parameters c, d, e and f satisfy the aforesaid ranges, the 2:1 type layered clay mineral can then be kept intact. The polyvalent cations selected from the aforesaid group are positioned between the layers, making sure enhanced caffeine adsorption capability.

Referring further to Fig. 1(A) and Fig. 1(B), a part or the whole of monovalent cations is replaced by polyvalent cations so that the cation density between the layers becomes low, leading to an enlargement of a space 150 between the cations positioned between the layers. In other words, the inner area of a two-dimensional layer occupied by one cation grows much larger as compared with a case where the cations positioned between the layers are all monovalent. Thus, the space 150 is so enlarged that the inner area of a two-dimensional layer occupied by one cation is enlarged, leading to accelerated caffeine adsorption. For the purpose of creating the enlarged space 150, the polyvalent cations are provided as the aforesaid E1 cations. Further, if the monovalent cations between the layers are replaced by polyvalent cations E1^{m+}, it is also expectable that the polarization of water coordinated at the polyvalent cations allows the layered clay mineral to develop strong solid acidity so that caffeine adsorption is further accelerated by way of interactions with the carbonyl groups of caffeine.

The inventive 2:1 type layered clay mineral 110 is preferably at least one smectite selected from the group consisting of montmorillonite, beidellite, saponite, hectorite and stevensite. These smectites are not only excellent in caffeine adsorption capability but may also be easily produced by ion exchange of easily available starting materials. Note here that smectites may be either natural minerals or synthetic materials.

Preferably, the inventive 2:1 type layered clay mineral 100 satisfies 0.2≦a+b≦0.6. That is, if the inventive 2:1 type layered clay mineral 110 has a layer charge of no less than 0.2 to no greater than 0.6, it is then more capable of caffeine adsorption.

On the other hand, 2:1 type layered clay minerals having a layer charge of 0.6 to 1.0 include vermiculites, mica clay minerals represented by illite, sericite, glauconite and celadonite, and mica minerals represented by phlogopite, biotite, muscovite, paragonite, taeniolite and tetrasilicon mica. Although these minerals provide a sheet having a larger area because their a- and b-axis direction crystallinity is higher than those of smectite crystals, they are less likely to provide the inventive 2:1 type layered clay mineral because of having often non-exchangeable potassium ions between the layers. Mica having interlayer ions replaced by hydrophilic ions such as sodium or lithium ions is known to swell limitedly by one or two water molecules, but it is likely to give rise to deterioration of caffeine adsorption capability thanks to its too high a charge density.

The layer charge of a 2:1 type layered clay mineral may be estimated from the results of the cation exchange capacity (CEC) of the layered compound measured by methods such as a column permeation method (see "Clay Handbook", 2nd Edition, edited by the Clay Science Society of Japan and published by Gihodo Shuppan, pp. 576 to 577) or a methylene blue adsorption method (Japan Bentonite Manufacturers Association Standard, JBAS-107-91). The layer charge may also be estimated by a method for analyzing the chemical composition of a clay mineral such as an X-ray fluorescent analysis, an energy dispersive X-ray (EDX) analysis making use of an electron microscope, or inductively coupled plasma optical emission spectrometry (ICP-OES) of a sample dissolved in acids or alkalis. In addition, the lattice constant of the layered clay mineral may be determined from the results of a structural analysis by electron beam diffraction using a transmission electron microscope or a structural analysis by the Rietveld method of powder x-ray diffraction, and then combined with the results of chemical composition analysis to figure out the cation density between the layers.

While the inventive 2:1 type layered clay mineral 110 has a layer charge of no less than 0.2 to less than 0.75, preferably no less than 0.2 to no greater than 0.6 and contains a polyvalent cation E1^{m+} at least between the layers, as described above, it is more preferable that 60% or greater of the layer charges are replaced by the polyvalent cations E1^{m+}. It is here noted that the replacement of no less than 60% of the layer charges by the polyvalent cations E1^{m+} is synonymous with the satisfaction of 0.12≦a<0.75, or 0.12≦a≦0.6 by the parameter a in the aforesaid composition formula. In the present invention, parameter a fulfills 0.12^a^0.6. This in turns ensures that, as shown schematically in Fig. 1(B), the cation density becomes low and the space 150 grows efficiently large so that there can be high caffeine adsorption capability achieved.

More preferably, the inventive 2:1 type layered clay mineral 110 has a layer charge of no less than 0.2 to no greater than 0.6 and, an occupied area per polyvalent cation E1^{m+} on a layer plane, in relation to the cation density, is no less than 0.8 nm²/cation to no greater than 9 nm²/cation. As the aforesaid occupied area is no less than 0.8 nm²/cation, it ensures that the space 150 for the adsorption of caffeine is of size large enough to make sure even more enhanced caffeine adsorption capability. As the cation density is no greater than 9 nm²/cation, it causes charges to be well balanced so that other cations are less likely to be taken in between the layers and, hence, a lowering of caffeine adsorption capability or a lowering of the separability by filtration due to interlayer swelling is less likely to take place. More preferably, the aforesaid occupied area is in a range of no less than 0.9 nm²/cation to no greater than 4 nm²/cation, in which much more enhanced caffeine adsorption capability is obtained together with high separability by filtration.

In the inventive 2:1 type layered clay mineral 110, the polyvalent cation E1^{m+} has a strong interaction with the 2:1 layer 130 so that when it turns into a bulky hydrated cation by way of the coordination of a water molecule, it plays a pillar-like role of keeping a layer spacing while enlarging it. In the inventive 2:1 type layered clay mineral, consequently, the polyvalent cation E1^{m+} acting as the pillar makes it possible to keep a constant layer spacing without giving rise to infinite interlayer swelling or delamination even in water. In other words, even when the inventive 2:1 type layered clay mineral is put in water, the strong interaction of the polyvalent cation E1^{m+} with the tetrahedral sheet and water molecule is unlikely to make the layer spacing larger as compared with the powder sample before put in water. This layer spacing, a value figured out of the peak position of the (001) reflection as measured by X-ray diffraction, is preferably maintained in a range of no less than 1.2 nm to no greater than 1.9 nm, in which range the aforesaid space 150 remains enlarged, making sure enhanced caffeine adsorption capability and enhancing the separability by filtration.

Among factors having an influence on caffeine adsorption capability there is a primary particle diameter of the 2:1 type layered clay mineral 110. The inventive 2:1 type layered clay mineral 110 has a mean primary particle diameter of preferably no less than 10 nm to no greater than 2 µm, and more preferably no less than 20 nm to no greater than 2 µm. A 2:1 type layered clay mineral having a mean primary particle diameter of no less than 10 nm is easy to produce and refine, and excels in handling as well. Setting the mean primary particle diameter at no greater than 2 µm, on the other hand, makes the specific surface area high enough to render the caffeine adsorption efficiency high.

A value as measured using a transmission electron microscope (TEM) image, a scanning electron microscope (SEM) image or a scanning probe microscope (SPM) image is adopted as the mean primary particle diameter of the inventive 2:1 type layered clay mineral 110. When a sample that has been agglutinated and consolidated into a secondary particle is measured, the sample is preferably separated and dispersed to primary particles for measurement.

The particle diameter is measured as the constant direction diameter (Green diameter) in a direction vertical to the c-axis (the ab-plane) of the layered silicate crystal forming primary particles, and the particle diameters measured are then calculated as a number average to figure out a mean primary particle diameter.

In the inventive 2:1 type layered clay mineral 110, the aforesaid polyvalent cation E1^{m+} is preferably at least one selected from the group consisting of Mg, Al, Si, Sc, Ca, Ti, V, Cr, Mn, Fe, Ni, Cu, Ga and Zr. These polyvalent cations are likely to turn into bulky hydrated cations because of having high hydration energy. The interposition of these exchangeable polyvalent cations intensifies an electrical attraction between the tetrahedral sheets and, hence, elicits an effect on maintaining a limited swelling state in which the basal plane spacing becomes narrower than 4 nm. In turn, this makes the inventive 2:1 type layered clay mineral 110 likely to keep the aforesaid space 150 while it remains enlarged, making sure more enhanced caffeine adsorption capability and enhancing the separability by filtration.

Referring here to Fig. 1(C), the disclosed caffeine adsorption material may contain a derivative 120 derived from the inventive 2:1 type layered clay mineral (Fig. 1(B)) (hereafter called the derivative for short). The derivative 120 is obtained on the basis of the inventive 2:1 type layered clay mineral 110, and has an interlayer pillar 160 derived from polyvalent cations. This pillar 160 then makes the cation density so low and the space 150 so large that the
derivative 120, too, has caffeine adsorption capability.

More specifically, the polyvalent cation E1^{m+} positioned between layers as described above turns into a bulky hydrated cation by way of coordination of a water molecule and plays a pillar-like role of enlarging an interlayer spacing. Further, this polyvalent cation deprives the coordinated water molecule of OH⁻ (viz., by hydrolysis) to form a difficult-to-dissolve hydroxide (hereafter called the metal hydroxy complex) providing a pillar 160. A layered compound containing the aforesaid pillar 160 may be used as a favorable porous material. A layered compound containing the pillar 160 in the form of an oxide or its crosslinked product obtained by firing of a layered compound containing such a metal hydroxy complex may also be included in the derivative 120.

The polyvalent cation E1^{m+} that forms the metal hydroxy complex is preferably provided by a small-sized ion having a large charge. Preferred among the aforesaid cations E1^{m+} is a cation having an ion radius-to-charge z ratio or, in another parlance, log (|z|/r), of +0.40 to +1.1 and an electronegativity of 1.2 to 1.9. Specifically, E1^{m+} is preferably a polyvalent cation of at least one element selected from the group consisting of Al, Si, Sc, Ti, V, Mn, Fe, Ni, Cu and Ga. With such polyvalent cations, the metal hydroxy complex could be generated with ease and stabilized in the form of the interlayer pillar 160. That is, with these polyvalent cations, the coordinating water molecule would easily be deprived of OH⁻ to form a slightly soluble hydroxide by way of pH changes of an interlayer environment, valence variations of metal ions or the like during repeated washing and drying. By contrast, Na ions, K ions, etc. are easy to dissolve in water because the ion radius-to-charge ratio (log (|z|/r)) is <+0.40 and the electronegativity is <1.2, whereas ions that are of small size but have a large charge (log (|z|/r is >+1.1 and an electronegativity of >1.9) are again easy to dissolve in water because they react with water and change into large-sized oxo anions (for instance, SO₄²⁻), resulting in spreading of charges in a large space.

Also, a polynuclear composite hydroxide cation based on the polyvalent cation E1^{m+} functions as the pillar 160 having interlayer stability; a layered compound containing this between layers, too, is included in the
derivative 120. The polyvalent cation E1^{m+} that forms such a polynuclear composite hydroxide cation is preferably a polyvalent cation of at least one element selected from the group consisting of Al, Si, Cr, Fe, Ga and Zr. More specifically, the polynuclear composite hydroxide cation is one or more composite ions selected from the group consisting of [Al₁₃O₄(OH)₂₄]⁷⁺, [Ga₁₃O₄(OH)₂₄]⁷⁺, [GaO₄Al₁₂(OH)₂₄]⁷⁺, [Zr₄(OH)₁₄]²⁺, [Fe₃O(OCOCH₃)_{6]}+, [Feₙ(OH)ₘ]^{3n-m} where 2≦m≦10 and 2≦n≦4 are satisfied, [Crₙ(OH)ₘ]^{3n-m} where 5≦m≦14 and 2≦n≦4 are satisfied, [ZrOCl₂-Al₈(OH)₂₀]⁴⁺ and [Al₁₃O₄(OH)₂₄₋ₙ]-[OSi(OH)₃]ₙ⁷⁺ where 1≦n≦23 is satisfied. Further, a layered compound containing a pillar 160 in the form of an oxide or its crosslinked product obtained by firing of a layered compound containing such a polynuclear composite hydroxide cation may also be included in the derivative 120.

It goes without saying that the derivative 120 should preferably satisfy the aforesaid occupied area on the layer plane, basal plane spacing and primary particle diameter as is the case with the inventive 2:1 type layer clay mineral.

As described above, the inventive 2:1 type layered clay mineral 110 makes it possible to keep a limited swelling state without incurring infinite swelling or delamination by containing the given polyvalent cation E1^{m+}, metal hydroxy complex based thereon, polynuclear composite hydroxide cation, or oxide or its crosslinked product between the layers of the 2:1 type layered clay mineral having a predetermined layer charge. In turn, this makes the space 150 for adsorption of caffeine intentionally larger as compared with the conventional 2:1 type layered clay mineral (100 in Fig. 1(A)) thereby keeping a limited swelling state where there is neither infinite swelling nor delamination, resulting in a remarkable improvement in caffeine adsorption capability in a low concentration region and high separability by filtration.

While the inventive caffeine adsorption material comprises the aforesaid inventive 2:1 type layered clay mineral 110 as the main ingredient, it is understood that it may further contain at least one additive selected from the group consisting of active carbon, acid clay, activated clay and zeolite. This makes the caffeine adsorption efficiency much higher. It is here understood that the amount of the main ingredient: the inventive 2:1 type layered clay mineral 110 is preferably no less than 50 wt% in view of caffeine adsorption capability. With the proviso that the caffeine adsorption material of the invention comprises the inventive 2:1 type layered clay mineral 110 as the main ingredient, it may further be mixed with a polyphenol adsorbent such as polyvinylpyrrolidone (PVPP) other than the aforesaid additive for simultaneous removal of caffeine and polyphenol.

The inventive caffeine adsorption material may be a composite or mixture comprising the inventive 2:1 type layered clay mineral 110 shown in Fig. 1(B) and its derivative 120 shown in Fig. 1(C). For instance, when Al is selected as E1, Al is likely to generate the metal hydroxy complex so that both Al³⁺ ion and hydroxy complex of Al may be contained between the layers. This embodiment, too, is included in the scope of the invention.

An exemplary process of producing the inventive caffeine adsorption material will now be explained.

The caffeine adsorption material comprising the inventive 2:1 type layered clay mineral 110 as the main ingredient, as shown in Fig. 1(B), may be produced using an existing ion exchange method. For instance, any 2:1 type layered clay mineral having a layer charge of no less than 0.2 to less than 0.75 may be used as the starting material, and then added to a salt solution of polyvalent cations E1^{m+}, followed by stirring, filtration and washing.

When the starting material contains extremely large or small primary particles, the large particles may be finely divided by pulverization using a ball mill, a jet mill or the like, or the small particles may be separated by levigation for the purpose of enhancing the ability of the inventive 2:1 type layered clay mineral 110 to adsorb caffeine.

For the production of a caffeine adsorption material comprising as the main ingredient the derivative 120 of the 2:1 type layered clay mineral 110 shown in Fig. 1(C) in the form of a layered compound containing the metal hydroxy complex of E1^{m+} between layers, on the other hand, the selection of E1^{m+} likely to generate the metal hydroxy complex is all that is needed for the production of the inventive 2:1 type layered clay mineral 110. For the production of a caffeine adsorption material containing the polynuclear composite hydroxide cations of E1^{m+} between layers as the main ingredient, E1 likely to generate polynuclear composite hydroxide cations may be selected and a salt solution of those cations may then be used.

Further, for the production of a caffeine adsorption material composed mainly of a layered compound containing an oxide or its crosslinked product as the derivative 120 of the inventive 2:1 layered clay mineral 110 shown in Fig. 1(C), the layered compound containing a metal hydroxy complex or polynuclear composite hydroxide cations may be heated in a temperature range of no less than 300°C to no greater than 800°C, whereby the formation of the oxide or cross-linked product is accelerated to make the interlayer pillar 160 more stable. Too low a heating temperature may possibly cause the reaction to proceed insufficiently, whereas too high a heating temperature may possibly incur destruction of the crystal structure of the layered clay mineral.

### Mode 2 of the Invention

In Mode 2 of the invention, the applications of the caffeine adsorption material described with reference to Mode 1 will be explained. The caffeine adsorption material of the invention is well suited for the selective adsorption of caffeine, and may be applied to filters and column fillers.

A filter using the inventive caffeine adsorption material comprises the inventive caffeine adsorption material carried on a fibrous material. A matrix for an existing filter may be used for the fibrous material. Such a matrix is exemplified by woven fabric, knitted fabric, nonwoven fabric or the like, among which the nonwoven fabric is preferably used. Such a fibrous material is exemplified by such a thermoplastic polymer as represented by polyolefin, polyamide and polyester. While the inventive caffeine adsorption material is carried on the fibrous material, it is understood that a binder or the like may be used if required. The caffeine adsorption material may be carried on the fibrous material preferably in an amount of no less than 20% by mass to no greater than 80% by mass.

A column filler using the inventive caffeine adsorption material contains granules obtained by the granulation of the inventive caffeine adsorption material. Granulation may be conducted by methods such as pelletizing, and evaporation spraying. The granules have a granule diameter of no less than 1 µm to no greater than 5 nm.

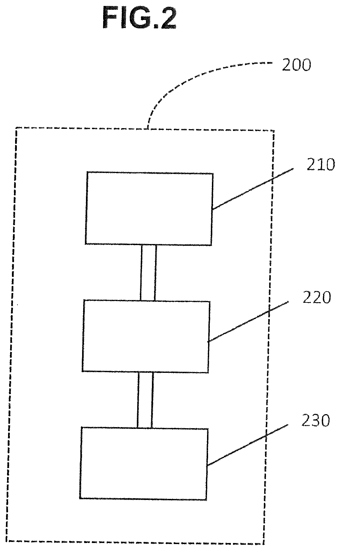

Fig. 2 is a schematic view of the inventive caffeine removal system.

A caffeine removal system comprising a filter or column filler using the inventive caffeine adsorption material is explained with reference to Fig. 2. A caffeine removal system 200 comprises a feeding means 210 for feeding a caffeine-containing fluid, a removal means 220 for selective removal of caffeine from the caffeine-containing fluid, and a recovery mean 230 for recovering the fluid out of which caffeine has been removed. The aforesaid caffeine adsorption filter or caffeine adsorption column filler is used for the removal means 220.

As the caffeine-containing fluid is fed to the removal means 220 through the feeding means 210, it causes the inventive caffeine adsorption material to come into contact with the caffeine-containing fluid for selective adsorption of caffeine. Passing through the removal means 220 where caffeine is removed, the caffeine-free fluid is recovered at the recovery means 230.

If the inventive caffeine adsorption material is used, it is then possible to provide selective adsorption and removal of caffeine even in a low concentration region because it excels in caffeine selectivity. With the inventive caffeine adsorption material capable of keeping the limited swelling state, the separability by filtration is so enhanced that the process steps can be simplified with a shortening of filtration time.

It is here noted that the inventive caffeine adsorption material may be used alone without being processed to any filter or column for adsorption and centrifugal separation of caffeine from the caffeine-containing fluid.

By way of example but not by way of limitation, the modes for carrying out the invention will be explained in further details with reference to examples and comparative examples.

### Examples

### Estimation Methods

How to form estimations in the invention is explained.

### 1. Layer Charge of the Layered Clay Mineral

The layered clay mineral was dissolved by an acid or alkali, and inductively coupled plasma optical emission spectrometry (ICP-OES, SPS3520UV-DD made by Hitachi High -Technologies) was then used to determine a composition formula of the clay mineral from the results of composition analysis for estimation of the absolute value of negative charge per unit.

### 2. Measurement of the Amount of Caffeine Adsorbed

As a result of determination of an adsorption isotherm from adsorption experimentation where aqueous solutions of caffeine in various concentrations were brought into contact with montmorillonite occurring in Yamagata Prefecture in a liquid-to-solid ratio of 100, it was when the solution concentration was 1.5 mmol/L that the highest adsorption rate was observed: 57% of caffeine was adsorbed onto montmorillonite. With this in mind, a caffeine aqueous solution having a concentration of 1.5 mmol/L was used for experimentation under the same conditions as mentioned above, where an adsorption material having 70% or higher of caffeine adsorbed onto it was judged as being acceptable. In the adsorption processing, 0.3 gram of clay mineral and 30 mL of a caffeine aqueous solution having a concentration of 1.5 mmol/L were added to a polypropylene (PP) centrifuge tube in which they were stirred at 50 rpm and 23°C for 24 hours using a overturning shaker (Rotor Mix RKVSD). Thereafter, the solution was centrifugally separated (at 10000 rpm for 5 minutes) into a liquid and a solid, followed by filtration of a solution component by a filter having a pore diameter of 0.2 µm. The resultant filtrate was set in a cell having an optical path length of 2 cm to measure the concentration of caffeine using a UV-visible spectrophotometer. A UV-visible spectrophotometer (UV-2450 made by Shimadzu Corporation) was used to prepare a calibration curve in advance from the maximum adsorption of caffeine near 275 nm, and the concentration of caffeine in a supernatant was estimated according to Lambert's law to calculate the rate of adsorption of caffeine onto the clay mineral.

### 3. Separability by filtration (Permeability)

A centrifugal separator was used for solid/liquid separation under the conditions of 10000 rpm and 5 minutes and, thereafter, a supernatant (about 30 mL) was filtrated through a disposable syringe provided at an end with a filter having a pore diameter of 0.2 µm. The results of estimation of separability by filtration are set out below.

### Estimation

○: The supernatant can be easily filtrated.
△: The filter is clogged up by a clay component remaining in the supernatant, so that filtration cannot be carried through, leaving a slight solution in the syringe.
×: 15 mL or more of the supernatant cannot be filtrated thanks to the clogging of the filter.

### Example 1

A caffeine adsorption material containing montmorillonite (E1 was Al) as the 2:1 type layered clay mineral was prepared in Example 1.

Na-type montmorillonite (Kunipia F made by KUNIMINE INDUSTRIES CO., LTD.) occurring in Yamagata Prefecture was used as the starting clay mineral. The aforesaid montmorillonite had a chemical composition:
(Na_{0.44}Ca_{0.03})[(Al_{1.56}Mg_{0.32}Fe_{0.10}Ti_{0.01})(Si_{3.85}Al_{0.15})O₁₀][(OH)_{1.98}F_{0 .02}] and a layer charge of 0.5. The mean Green diameter of the primary particles was 400 nm as measured under an electron microscope. One hundred (100) mL of an AlCl₃ aqueous solution having a concentration of 10 mmol/L were prepared as a processing agent used for modification (ion exchange) of the clay mineral. Then, 1 gram of the aforesaid montmorillonite in a dry state was added to the aqueous solution and sufficiently stirred at room temperature, followed by repeated filtration and washing. Note here that the concentration of this AlCl₃ aqueous solution was determined for the purpose of replacing 100% of interlayer Na with Al.

The thus modified clay mineral was dried in a hot-air dryer of 100°C, and both a dry powder sample and a sample wetted by water were then examined by an X-ray diffraction device (ULTIMA-IV made by Rigaku) in terms of reflection from the basal plane of the layered clay mineral. As a result of observation of the 001 reflection peak at 1.43 nm from both the samples, it has been identified that the samples capable of stable limited swelling are obtained. Note here that the Green diameter of the post-modification clay mineral was the same as before modification, as measured under an electron microscope.

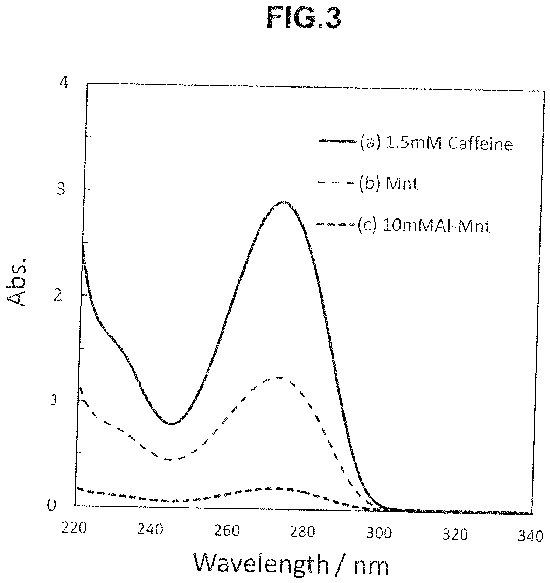

After caffeine adsorption processing, a solid component precipitated so very well that solid-liquid separation was easily achievable with the use of a 5-minutes centrifugal separation at 10000 rpm. A solution component was filtrated through a filter having a pore diameter of 0.2 µm for measurement of the concentration of caffeine in the filtrate by a UV-visible spectrophotometer. Fig. 3 is indicative of the UV-vis spectra of an aqueous solution having a caffeine concentration of 1.5 mmol/L and a supernatant after adsorption processing.

Fig. 3 is indicative of the UV-vis spectrum of the supernatant in the sample of Example 1 after adsorption processing.

In addition to the UV-vis spectrum (c) of the supernatant in the sample of Example 1 after adsorption processing, Fig. 3 shows the UV-vis spectrum (a) of a caffeine aqueous solution (1.5 mmol/L) and the UV-bis spectrum (b) of the supernatant after adsorption processing with unmodified montmorillonite.

According to Fig. 3, it has been shown that caffeine is selectively removed by use of the sample of Example 1. Based on the calibration curve, the present sample was capable of absorbing 91.2% of caffeine in the solution. The solid component precipitated well after caffeine absorption processing, and the filter filtration of the supernatant after a 5-minutes centrifugal separation at 10000 rpm could be performed without suffering from clogging due to an unprecipitated clay component. These results are set out in Table 2.

### Example 2

A caffeine adsorption material containing saponite (where E1 was Al) as the 2:1 type layered clay mineral was prepared in Example 2.

Much the same processing as in Example 1 was performed with the exception of changing the Na-type montmorillonite to synthetic saponite (Smecton-SA made by KUNIMINE INDUSTRIES CO., LTD.). The starting synthetic saponite had a chemical composition:
Na_{0.45}(Mg_{3.11})(Si_{3.53}Al_{0.40})O₁₀(OH)₂ and a layer charge of 0.45. The starting material has a mean particle diameter (Green diameter) of 35 nm as measured under an electron microscope. A powder sample dried in a hot-air dryer of 100°C after modification processing had a basal plane spacing of 1.50 nm, and from the results of caffeine adsorption testing, it was found that the aforesaid powder sample was capable of adsorbing 98.5% of caffeine in the solution. After the caffeine adsorption processing, a solid component precipitated very well, and the filter filtration of a supernatant after a 5-minutes centrifugal separation at 10000 rpm could be performed without suffering from clogging due to an unprecipitated clay component. These results are set out in Table 2.

### Example 3

A caffeine adsorption material containing montmorillonite (where E1 was Fe) as the 2:1 type layered clay mineral was prepared in Example 3.

Much the same processing as in Example 1 was performed with the exception that one hundred (100) mL of a FeCl₂ aqueous solution having a concentration of 10 mmol/L were added as a processing agent to the Na-type montmorillonite occurring in Yamagata Prefecture and used in Example 1, and the resultant solution was stirred while subjected to nitrogen bubbling for reactions. From the fact that the color of a powder sample dried in a hot-air dryer of 100°C changed to brown, a part of Fe²⁺ adsorbed between the layers or onto the surface was considered to be oxidized into Fe³⁺. The aforesaid powder sample had a basal plane spacing of 1.49 nm, and from the results of caffeine adsorption testing, it was found that the aforesaid powder sample was capable of adsorbing 93.5% of caffeine in the solution. After the caffeine adsorption processing, a solid component precipitated very well, and the filter filtration of a supernatant after a 5-minutes centrifugal separation at 10000 rpm could be performed without suffering from clogging due to an unprecipitated clay component. These results are set out in Table 2.

### Example 4

A caffeine adsorption material containing hectorite (where E1 was Fe) as the 2:1 type layered clay mineral was prepared in Example 4.

Much the same processing as in Example 3 was performed with the exception of changing the Na-type montmorillonite occurring in Yamagata Prefecture and used in Example 3 to synthetic hectorite (BYK, LAPONITE RD). The synthetic hectorite having a chemical composition: (Na_{0.37}Ca_{0.01})(Mg_{2.80}Li_{0.19})Si_{3.96}O₁₀(OH)₂ and a layer charge of 0.39. The mean primary particle diameter (Green diameter) was 30 nm as measured under an electron microscope. From the fact that the color of a powder sample dried in a hot-air dryer of 100°C changed to brown after the modification processing, a part of Fe²⁺ adsorbed between the layers or onto the surface was considered to be oxidized into Fe³⁺. The aforesaid powder sample had a basal plane spacing of 1.39 nm, and the aforesaid powder sample was capable of adsorbing 98.9% of caffeine in the solution. After the caffeine adsorption processing, a solid component precipitated very well, and the filter filtration of a supernatant after a 5-minutes centrifugal separation at 10000 rpm could be performed without suffering from clogging due to an unprecipitated clay component. These results are set out in Table 2.

### Example 5

A caffeine adsorption material containing montmorillonite (where E1 was Al) as the 2:1 type layered clay mineral was prepared in Example 5.

Much the same processing as in Example 3 was performed with the exception that the processing agent in Example 1 was changed to 100 mL of an AlCl₃ aqueous solution having a concentration of 3 mmol/L. This concentration of the AlCl₃ aqueous solution was determined for the purpose of replacing 67% of the layer charge with Al.

A powder sample dried in a hot-air dryer of 100°C had a basal plane spacing of 1.32 nm, and was capable of adsorbing 71.7% of caffeine in the solution. After the caffeine adsorption processing, a solid component precipitated very well, and the filter filtration of a supernatant after a 5-minutes centrifugal separation at 10000 rpm could be performed without suffering from clogging due to an unprecipitated clay component. These results are set out in Table 2.

### Example 6

A caffeine adsorption material containing montmorillonite (where E1 was Fe) as the 2:1 type layered clay mineral was prepared in Example 6.

Much the same processing as in Example 3 was performed with the exception that the processing agent in Example 3 was changed to 100 mL of a FeCl₂ aqueous solution having a concentration of 5 mmol/L. This concentration of the FeCl₂ aqueous solution was determined for the purpose of replacing 75% of the layer charge with Fe.

From the fact that the color of a powder sample dried in a hot-air dryer of 100°C changed to slight brown after the modification processing, a part of Fe²⁺ adsorbed between the layers or onto the surface was considered to be oxidized into Fe³⁺. As a result of examination of reflection from the basal plane of the aforesaid powder sample by an X-ray diffraction device, there were two peaks observed: 1.24 nm and 1.43 nm. As a result of caffeine adsorption testing, the aforesaid powder sample was found to be capable of adsorbing 90.2% of caffeine in the solution. After the caffeine adsorption processing, a solid component precipitated very well, and the filter filtration of a supernatant after a 5-minutes centrifugal separation at 10000 rpm could be performed without suffering from clogging due to an unprecipitated clay component. These results are set out in Table 2.

### Example 7

A caffeine adsorption material containing montmorillonite (where E1 was Fe) as the 2:1 type layered clay mineral was prepared in Example 7.

Much the same processing as in Example 3 was performed with the exception that the processing agent in Example 3 was changed to 100 mL of a FeCl₂ aqueous solution having a concentration of 100 mmol/L. This concentration of the FeCl₂ aqueous solution was determined for the purpose of performing the processing with too much Fe in excess of 100% of the layer charge.

From the fact that the color of a powder sample dried in a hot-air dryer of 100°C changed to brown after the modification processing, a part of Fe²⁺ adsorbed between the layers or onto the surface was considered to be oxidized into Fe³⁺. The aforesaid powder sample had a basal plane spacing of 1.56 nm. As a result of caffeine adsorption testing, the aforesaid powder sample was found to be capable of adsorbing 95.8% of caffeine in the solution. After the caffeine adsorption processing, a solid component precipitated very well, and the filter filtration of a supernatant after a 5-minutes centrifugal separation at 10000 rpm could be performed without suffering from clogging due to an unprecipitated clay component. These results are set out in Table 2.

### Reference Example 8

A caffeine adsorption material containing a montmorillonite derivative (where E1 was Al) containing a polynuclear composite hydroxide cation as the 2:1 type layered clay mineral derivative was prepared in Example 8.

A suspension (200 mL) containing 1% by mass of the clay used in Example 1 was prepared. Apart from this, a NaOH solution (500 mL) having a concentration of 0.4 mol was slowly added to an AlCl₃ solution (250 ml) having a concentration of 0.4 mmol while stirred and, thereafter, they were refluxed until the resulting solution remained transparent to obtain a transparent solution of polynuclear aluminum hydroxide ions [Al₁₃O₄(OH)₂₄]⁷⁺, each having a Keggin structure. The aforesaid suspension (200 mL) having a clay concentration of 1% by mass was slowly added to this transparent solution, followed by stirring for several hours. The resulting product was repeatedly filtrated and washed, then dried at 100°C and then pulverized into a powdery sample. As a result of examination of reflection from the basal plane of the obtained powdery sample, a peak of 1.82 nm and a shoulder reflection of 1.55 nm were observed. As a result of estimating the present sample in the same manner as in Example 1, it was capable of adsorbing 88.8% of caffeine in the solution. After the caffeine adsorption processing, a solid component precipitated very well, and the filter filtration of a supernatant after a 5-minutes centrifugal separation at 10000 rpm could be performed without suffering from clogging due to an unprecipitated clay component. These results are set out in Table 2.

### Example 9

A caffeine adsorption material containing montmorillonite (where E1 was Mg) as the 2:1 type layered clay mineral was prepared in Example 9.

Much the same processing as in Example 1 was performed with the exception of changing the processing agent used in Example 1 to MgCl₂. After the modification processing, a powder sample dried in a hot-air dryer of 100°C had a basal plane spacing of 1.55 nm. As a result of caffeine adsorption testing, the aforesaid powder sample was capable of adsorbing 90.6% of caffeine in the solution. After the caffeine adsorption processing, a solid component precipitated very well, and the filter filtration of a supernatant after a 5-minutes centrifugal separation at 10000 rpm could be performed without suffering from clogging due to an unprecipitated clay component. These results are set out in Table 2.

### Example 10

A caffeine adsorption material containing montmorillonite (where E1 was Ca) as the 2:1 type layered clay mineral was prepared in Example 10.

Much the same processing as in Example 1 was performed with the exception of changing the processing agent used in Example 1 to CaCl₂. After the modification processing, a powder sample dried in a hot-air dryer of 100°C had a basal plane spacing of 1.53 nm. As a result of caffeine adsorption testing, the aforesaid powder sample was capable of adsorbing 92.2% of caffeine in the solution. After the caffeine adsorption processing, a solid component precipitated very well, and the filter filtration of the supernatant after a 5-minutes centrifugal separation at 10000 rpm could be performed without suffering from clogging due to an unprecipitated clay component. These results are set out in Table 2.

### Reference Example 11

A caffeine adsorption material containing a saponite derivative (where E1 was Al) with an oxide between the layers as the 2:1 type layered clay mineral derivative was prepared in Example 11.

The sample obtained in Example 2 was heated at 500°C for 3 hours to make a similar estimation as in Example 2. As a result of examination of reflection from the basal plane of the obtained powder sample by an X-ray diffraction device, a broad reflection was observed in the vicinity of 1.20 nm, and a background rise was observed on an angle side lower than that reflection. As a result of caffeine adsorption testing, the aforesaid powder sample was capable of adsorbing 97.6% of caffeine in the solution. After the caffeine adsorption processing, a solid component precipitated very well, and the filter filtration of the supernatant after a 5-minutes centrifugal separation at 10000 rpm could be performed without suffering from clogging due to an unprecipitated clay component. These results are set out in Table 2.

### Comparative Example 1

For the purpose of estimation, much the same processing as in Example 1 was performed with the exception that the Na-type montmorillonite of Example 1 occurring in Yamagata Prefecture was used without any modification processing. A powder sample had a basal plane spacing of 1.25 nm. After caffeine adsorption processing, a solid component precipitated so poorly that the filter filtration using a 0.2 µm pore could somehow be carried through after solid-liquid separation by a 15-minutes centrifugal separation at 15000 rpm, although some clogging took place. As a result of caffeine adsorption testing, the powder sample was capable of adsorbing 57.0% of caffeine in the solution. The results are set out in Table 2.

### Comparative Example 2

For the purpose of estimation, much the same processing as in Example 2 was performed with the exception that the synthetic saponite of Example 2 was used without any modification processing. A powder sample had a basal plane spacing of 1.19 nm. Because the precipitation of a solid component was not visibly observed after caffeine adsorption processing, solid-liquid separation was performed by a 20-minutes centrifugal separation at 18000 rpm. Thereafter, filter filtration using a 0.2 µm pore was performed. Since clogging took place immediately, the first-round solution passing through the filter was applied to caffeine analysis. Consequently, 45.0% of caffeine was adsorbed out of the solution. The results are set out in Table 2.

### Comparative Example 3

Much the same processing as in Example 1 was performed with the exception that the processing agent for the Na-type montmorillonite of Example 1 occurring in Yamagata Prefecture was changed to 100 mL of a KCl aqueous solution having a concentration of 20 mmol/L. After modification processing, a powder sample dried in a hot-air dryer of 100°C had a basal plane spacing of 1.23 nm. While a solid component precipitated well after caffeine adsorption processing, some unfiltrated solution (of the order of a few mL) was left over in the filter filtration process of a supernatant after a 5-minutes centrifugal separation at 10000 rpm. As a result of caffeine adsorption testing, the aforesaid powder sample was capable of adsorbing 67.7% of caffeine in the solution. The results are set out in Table 2.

### Comparative Example 4

Much the same processing as in Comparative Example 1 was carried out with the exception of using an acid clay (MIZUKA ACE made by Mizusawa Industrial Chemicals, Ltd.) in place of the Na-type montmorillonite of Comparative Example 1 occurring in Yamagata Prefecture. A powder sample had a basal plane spacing of 1.53 nm. Although a solid component precipitated well after adsorption processing, some unfiltrated solution (of the order of a few mL) was left over in the filter filtration process of a supernatant after a 5-minutes centrifugal separation at 10000 rpm. As a result of caffeine adsorption testing, the aforesaid powder sample was capable of adsorbing 54.7% of caffeine in the solution. The results are set out in Table 2.

### Comparative Example 5

Much the same processing as in Comparative Example 1 was performed with the exception of using an activated clay (GALLEON EARTH V2 made by Mizusawa Industrial Chemicals, Ltd.) in place of the Na-type montmorillonite of Comparative Example 1 occurring in Yamagata Prefecture. In terms of a peak intensity of reflection from the basal plane, a powder sample was lower than acid clay of Comparative Example 4, and has a basal plane spacing of 1.54 nm. Although a solid component precipitated well after adsorption processing, some unfiltrated solution (of the order of a few mL) was left over in the filter filtration process of a supernatant after a 5-minutes centrifugal separation at 10000 rpm. As a result of caffeine adsorption testing, the aforesaid powder sample was capable of adsorbing 48.8% of caffeine in the solution. The results are set out in Table 2.

### Comparative Example 6

Much the same processing as in Example 3 was performed with the exception that the acid clay (1 gram) used in Comparative Example 4 was added to 100 mL of a FeCl₂ aqueous solution having a concentration of 10 mmol/L for reactions in a nitrogen atmosphere. From the fact that the color of a powder sample dried in a hot-air dryer of 100°C changed to brown after modification processing, a part of Fe²⁺ adsorbed between the layers or onto the surface was considered to be oxidized into Fe³⁺. The powder sample had a basal plane spacing of 1.47 nm. A solid component precipitated well after caffeine adsorption processing, and the filter filtration of a supernatant after a 5-minutes centrifugal separation at 10000 rpm could be performed without suffering from clogging due to an unprecipitated clay component. As a result of caffeine adsorption testing, the powder sample was capable of adsorbing 63.1% of caffeine in the solution, but the adsorption goal (70% or greater) of the present invention could not be achieved. The results are set out in Table 2.

### Comparative Example 7

Much the same processing as in Example 3 was performed with the exception that the activated clay (1 gram) used in Comparative Example 5 was added to 100 mL of a FeCl₂ aqueous solution having a concentration of 10 mmol/L for reactions in a nitrogen atmosphere. From the fact that the color of a powder sample dried in a hot-air dryer of 100°C changed to brown after modification processing, a part of Fe²⁺ adsorbed between the layers or onto the surface was considered to be oxidized into Fe³⁺. The powder sample had a basal plane spacing of 1.48 nm. A solid component precipitated well after caffeine adsorption processing, and the filter filtration of a supernatant after a 5-minutes centrifugal separation at 10000 rpm could be performed without suffering from clogging due to an unprecipitated clay component. As a result of caffeine adsorption testing, the powder sample was capable of adsorbing just only 49.7% of caffeine in the solution; any enhanced adsorption capability by FeCl₂ processing could not be identified. The results are set out in Table 2.

### Comparative Example 8

A cycle in which 1 gram of a biotite occurring in China and having a chemical composition: (K_{0.91}Na_{0.05}Ca_{0.02})(Fe_{1.06}Mg_{1.46}Al_{0.275}Ti_{0.13}M_{n0.01})(Si_{2.84}Al_{1.16})O₁₀(O H)_{1.9}F_{0.1} and a layer charge of 1.0 was stirred in 200 mL of a 5M NaNO₃ aqueous solution at 90°C for 24 hours was repeated three times to prepare a Na-type biotite having a chemical composition: (Na_{0.75}K_{0.01}Ca_{0.01})(Fe_{1.07}Mg_{1.48}Al_{0.3}Ti_{0.13}Mn_{0.01})(Si_{2.86}Al_{1.14})O₁₀(OH )_{1.9}F_{0.1}. The Na-type biotite had a mean primary particle diameter (Green diameter) of 29.0 µm. For modification processing, 100 mL of an AlCl₃ aqueous solution having a concentration of 10 mmol/L were added to this Na-type biotite. As a result of examination of reflection from the basal plane of a powder sample dried in a hot-air dryer of 100°C by an X-ray diffraction device, the 001 reflection of a basal plane spacing of 1.43 nm was observed. As a result of the same caffeine adsorption testing performed as in Example 1, a solid component precipitated well, and the filter filtration of a supernatant after a 5-minutes centrifugal separation at 10000 rpm could be performed without suffering from clogging due to an unprecipitated clay component. The then caffeine adsorption rate was 2.7%. The results are set out in Table 2.

### Comparative Example 9

Much the same processing as in Example 3 was performed with the exception of using a Na-type synthetic fluoro-tetrasilicic mica made by Topy Industries, Ltd. with a chemical composition: Na_{0.75}Mg_{2.83}Si₄O₁₀F₂ and a layer charge of 0.75. The mean primary particle diameter (Green diameter) was 3.9 µm. From the fact that the color of a powder sample dried in a hot-air dryer of 100°C after processing with FeCl₂ changed to brown, a part of Fe²⁺ adsorbed between the layers or onto the surface was considered to be oxidized into Fe³⁺. As a result of examination of reflection from the basal plane of the obtained powder sample by an X-ray diffraction device, there were two 001 reflections of 1.46 nm and 1.50 nm observed. As a result of the same caffeine adsorption testing performed as in Example 1, a solid component precipitated well after the testing, and the filter filtration of a supernatant after a 5-minutes centrifugal separation at 10000 rpm could be carried out without suffering from clogging due to an unprecipitated clay component. The then caffeine adsorption rate was 2.0%. The results are set out in Table 2.

### Comparative Example 10

Much the same processing as in Example 3 was performed with the exception of using halloysite (reagent available from Aldrich) represented by a chemical formula Al₂Si₂O₅(OH)₄·2H₂O and having a layer charge of 0 as the starting clay mineral. From the fact that the color of a powder sample dried in a hot-air drier of 100°C after processing with FeCl₂ changed to brown, a part of Fe²⁺ adsorbed between the layers or onto the surface was considered to be oxidized into Fe³⁺. The powder sample had a basal plane spacing of 0.74 nm. In a filtration process after a 5-minutes centrifugal separation at 10000 rpm, a filter was clogged up, resulting in the inability to filtrate 15 mL or more of a supernatant. The powder sample had a caffeine adsorption rate of 5.7%. The results are set out in Table 2.

The experimental conditions for the foregoing examples and comparative examples are set out in Table 1, and the results of estimation of caffeine adsorption rates and separability by filtration (permeability) are set out in Table 2.

**Table 1: A listing of the experimental conditions for Examples 1 to 11 and Comparative Examples 1 to 10**

| Examples/ Comparative Examples | | Starting Clay Mineral | | |
|---|---|---|---|---|
| | | Name of the Mineral | Layer Charge | Mass (g) |
| | Example 1 | Montmorillonite | 0.5 | 1 |
| | Example 2 | Synthetic Saponite | 0.45 | 1 |
| | Example 3 | Montmorillonite | 0.5 | 1 |
| | Example 4 | Synthetic Hectorite | 0.39 | 1 |
| | Example 5 | Montmorillonite | 0.5 | 1 |
| | Example 6 | Montmorillonite | 0.5 | 1 |
| | Example 7 | Montmorillonite | 0.5 | 1 |
| | Example 8 | Montmorillonite | 0.5 | 1 |
| | Example 9 | Montmorillonite | 0.3 | 1 |
| | Example 10 | Montmorillonite | 0.3 | 1 |
| | Example 11 | Synthetic Saponite | 0.45 | 1 |
| Comparative Example 1 | | Montmorillonite | 0.5 | 1 |
| Comparative Example 2 | | Synthetic Saponite | 0.45 | 1 |
| Comparative Example 3 | | Montmorillonite | 0.5 | 1 |
| Comparative Example 4 | | Acid Clay | Unknown | 1 |
| Comparative Example 5 | | Activated Clay | Unknown | 1 |
| Comparative Example 6 | | Acid Clay | Unknown | 1 |
| Comparative Example 7 | | Activated Clay | Unknown | 1 |
| Comparative Example 8 | | Na-Type biotite | 1.0 | 1 |
| Comparative Example 9 | | Na-Type Synthetic fluoro-tetrasilicic mica | | |
| | | | 0.75 | 1 |
| Comparative Example 11 | | Halloysite | 0 | 1 |

| Conditions for Modification Processing | | | | |
|---|---|---|---|---|
| | E1 Aq. Solutions | | Drying Temp. (°C) | |
| Ex. 1 | AlCl₃ aq. (10 mM, 100 mL) | | 100 | |
| Ex. 2 | AlCl₃ aq. (10 mM, 100 mL) | | 100 | |
| Ex. 3 | FeCl₂ aq. (10 mM, 100 mL) | | 100 | |
| Ex. 4 | FeCl₂ aq. (10 mM, 100 mL) | | 100 | |
| Ex. 5 | AlCl₃ aq. (3 mM, 100 mL) | | 100 | |
| Ex. 6 | FeCl₂ aq. (5 mM, 100 mL) | | 100 | |
| Ex. 7 | FeCl₂ aq. (100 mM, 100 mL) | | 100 | |
| Ex. 8 | [Al₁₃O₄(OH)₂₄]⁷⁺ aq. | | 100 | |
| Ex. 9 | MgCl₂ aq. (10 mM, 100 mL) | | 100 | |
| Ex. 10 | CaCl₂ aq. (10 mM, 100 mL) | | 100 | |
| Ex. 11 | AlCl₃ aq. (10 mM, 100 mL) | | 500 | |
| CE. 1 | - | | - | |
| CE. 2 | - | | - | |
| CE. 3 | KCl aq. (20 mM, 100 mL) | | 100 | |
| CE. 4 | - | | - | |
| CE. 5 | - | | - | |
| CE. 6 | FeCl₂ aq. (10 mM, 100 mL) | | 100 | |
| CE. 7 | FeCl₂ aq. (10 mM, 100 mL) | | 100 | |
| CE. 8 | AlCl₃ aq. (10 mM, 100 mL) | | 100 | |
| CE. 9 | FeCl₂ aq. (10 mM, 100 mL) | | 100 | |
| CE. 10 | FeCl₂, aq. (10 mM, 100 mL) | | 100 | |
| CE: Comparative Example | | | | |

| After Modification | | | | |
|---|---|---|---|---|
| | E1^{m+} | E2⁺ | | |
| Example 1 | Al³⁺ | *Na⁺ | | |
| Example 2 | Al³⁺ | *Na⁺ | | |
| Example 3 | Fe²⁺, Fe³⁺ | *Na⁺ | | |
| Example 4 | Fe²⁺, Fe³⁺ | *Na⁺ | | |
| Example 5 | Al³⁺ | Na⁺ | | |
| Example 6 | Fe²⁺, Fe³⁺ | Na⁺ | | |
| Example 7 | Fe²⁺, Fe³⁺ | *Na⁺ | | |
| Example 8 | [Al₁₃O₄(OH)₂₄]⁷⁺ | Na⁺ | | |
| Example 9 | Mg²⁺ | *Na⁺ | | |
| Example 10 | Ca²⁺ | *Na⁺ | | |
| Example 11 | Al³⁺ | *Na⁺ | | |
| Comp. Ex. 1 | - | Na⁺ | | |
| Comp. Ex. 2 | - | Na⁺ | | |
| Comp. Ex. 3 | K⁺ | Na⁺ | | |
| Comp. Ex. 4 | - | H⁺ | | |
| Comp. Ex. 5 | - | H⁺ | | |
| Comp. Ex. 6 | Fe²⁺, Fe³⁺ | H⁺ | | |
| Comp. Ex. 7 | Fe²⁺, Fe³⁺ | H⁺ | | |
| Comp. Ex. 8 | Al³⁺ | Na⁺ | | |
| Comp. Ex. 9 | Fe²⁺, Fe³⁺ | Na⁺ | | |
| Comp. Ex. 10 | Fe²⁺, Fe³⁺ | - | | |

Referring to Table 1, it is noted that the asterisk (*) indicates that one specific object of the invention is to subject all Na⁺ to ion exchange thereby allowing all Na⁺ to disappear substantially but, in some instances, there may be unexchanged Na⁺ unavoidably left over.

From Table 1, each of the samples of Examples 1 to 7, 9 and 10 is the 2:1 type layered clay mineral containing at least between layers a polyvalent cation E1^{m+} or a metal hydroxy complex based thereon, and its derivative; the sample of Example 8 is a derivative of the 2:1 type layered clay mineral containing at least between layers a polynuclear composite hydroxide cation based on the polyvalent cation E1^{m+}; and the sample of Example 11 is a derivative of the 2:1 type layered clay mineral containing at least between layers an oxide wherein the hydroxy complex of the polyvalent cation E1^{m+} is dehydrated.

**Table 2: Results of the caffeine adsorption rate (%) and permeability of the samples according to Examples 1 to 11 and Comparative Examples 1 to 10**

| Examples/ Caffeine Adsorption Permeability | | |
|---|---|---|
| Comp. Examples | Rate (%) | |
| Example 1 | 91.2 | ○ |
| Example 2 | 98.5 | ○ |
| Example 3 | 93.5 | ○ |
| Example 4 | 98.9 | ○ |
| Example 5 | 71.7 | ○ |
| Example 6 | 90.2 | O |
| Example 7 | 95.8 | ○ |
| Example 8 | 88.8 | ○ |
| Example 9 | 90.6 | ○ |
| Example 10 | 92.2 | ○ |
| Example 11 | 97.6 | ○ |
| Comp. Ex. 1 | 57.0 | × |
| Comp. Ex. 2 | 45.0 | × |
| Comp. Ex. 3 | 67.7 | △ |
| Comp. Ex. 4 | 54.7 | △ |
| Comp. Ex. 5 | 48.8 | △ |
| Comp. Ex. 6 | 63.1 | ○ |
| Comp. Ex. 7 | 49.7 | ○ |
| Comp. Ex. 8 | 2.7 | ○ |
| Comp. Ex. 9 | 2.0 | ○ |
| Comp. Ex. 10 | 5.7 | × |

According to Examples 1 to 11 in Table 2, it has been shown that the 2:1 type layered clay minerals and their derivatives maintain the limited swelling state, have caffeine adsorption capability in a low concentration region and high separability by filtration, and function effectively as a caffeine adsorption material.

Referring in detail to Examples 1 to 11 and Comparative Example 10, it has been shown that the inventive caffeine adsorption material comprises the 2:1 type layered clay mineral as a layered clay mineral providing a basic structure, and referring in detail to Examples 1 to 11 and Comparative Examples 8 and 9, it has been shown that the inventive 2:1 type layered clay minerals meet a layer charge range of no less than 0.2 to less than 0.75. Referring further to Examples 1 to 11 and Comparative Examples 1 to 3, it has been shown that the inventive 2:1 type layered clay mineral contains the polyvalent cations E1^{m+} at least between layers.

Referring to Examples 1 to 11 and Comparative Examples 4 and 5, it has been shown that the inventive caffeine adsorption material is capable of selective adsorption of caffeine even in a low concentration region and has higher separability by filtration (permeability) as compared with acid clay and activated clay used as an existing caffeine adsorption material. Referring further to Comparative Examples 4 and 6 as well 5 and 7, respectively, it has been shown that even when acid clay or activated clay available as an existing caffeine adsorption material is processed for modification with the use of polyvalent cations, any desired adsorption rate is not obtained at all. The aforesaid Patent Publication 5 discloses that, as in Comparative Examples 6 and 7, acid clay or activated clay is processed with the use of a cation selected from the group consisting of a potassium ion, a calcium ion and a magnesium ion. However, Patent Publication 5 does not disclose whatsoever that these cations contribute to the content of caffeine, although some appearance or flavor is maintained. From these, it has been shown that even when acid clay or activated clay is processed for modification with the use of polyvalent cations E1^{m+}, the inventive 2:1 type layered clay mineral are not obtained, partly because the polyvalent cation is not ion exchanged or if it is done, the amount of ion exchange is insufficient; any remarkable enhancement in terms of caffeine adsorption capability would not be expectable.

From the examples and comparative examples described so for, it has been identified that the inventive 2:1 type layered clay mineral excels in caffeine adsorption capability. To identify that the inventive 2:1 type layered clay mineral is well capable of adsorption of purine bases other than caffeine, its adsorption capability for adenine that was one of purine bases was examined in the following example and comparative example.

The amount of adsorption of adenine was measured by the following method and procedure. 0.3 gram of clay mineral and 30 mL of an adenine aqueous solution having a concentration of 5 mmol/L were added to a polypropylene (PP) centrifuge tube in which they were stirred at 50 rpm and 23°C for 24 hours using a overturning shaker (Rotor Mix RKVSD). Thereafter, the solution was centrifugally separated (at 10000 rpm for 5 minutes) into a liquid and a solid, followed by filtration of a solution component by a filter having a pore diameter of 0.2 µm. The resultant filtrate was set in a cell having an optical path length of 2 cm to measure the concentration of adenine using a UV-visible spectrophotometer. A UV-visible spectrophotometer (UV-2450 made by Shimadzu Corporation) was used to prepare a calibration curve in advance from the maximum adsorption of adenine near 260 nm, and the concentration of adenine in a supernatant was estimated according to Lambert's law to calculate the rate of adsorption of adenine onto the clay mineral.

### Example 12

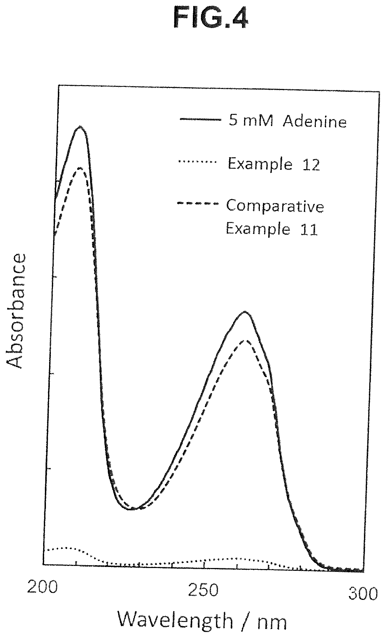

The post-modification montmorillonite used in Example 1 was used as the clay mineral. After adenine adsorption processing, a solid component precipitated very well, and solid-liquid separation could easily be performed by a 5-minutes centrifugal separation at 10000 rpm. The filter filtration of a solution component (supernatant) could also be conducted without suffering from clogging due to an unprecipitated clay component. Fig. 4 shows the UV-vis spectra of a filtrate. The clay mineral according to this example was capable of adsorbing 95% of adenine in the solution.

### Comparative Example 11

For examination of adenine adsorption capability, the same processing as in Example 12 was performed with the exception that the Na-type montmorillonite used as the starting material in Example 1 and occurring in Yamagata Prefecture was used as the clay mineral. Since a solid component precipitated poorly after adenine adsorption processing, solid-liquid separation was performed by a 15-minutes centrifugal separation at 15000 rpm and then filtration using a filter having a pore diameter of 0.2 µm was conducted. Consequently, filtration could be carried out, although there was some clogging observed. Fig. 4 shows the UV-vis spectra of a filtrate. As a result of adenine adsorption testing, the adenine adsorption rate of the Na-type montmorillonite remained at 11.20.

Referring to Example 12 and Comparative Example 11, it has been shown that the layered clay mineral according to the invention excels in terms of adsorption capability for not only caffeine but also other purine bases such as adenine.

### Industrial Applicability

Even when the inventive material for adsorption of purine bases is applied to an aqueous solution containing a purine base in a low concentration, it is possible to adsorb and separate the purine base effectively at lower costs. Such a purine base adsorption material is best suited for a purine base adsorption filter, a purine base adsorption column filler, and the same is applied to a purine base removal system.

### Explanation of the Reference Numerals

100: Existing 2:1 type layered clay mineral
110: Inventive 2:1 type layered clay mineral
120: Derivative of the inventive 2:1 type layered clay
mineral
130: 2:1 layer
140: Cation(s)
150: Space
160: Pillar(s)
200: Purine base removal system
210: Feeding means
220: Removal means
230: Recovery means

## Claims

1. A purine base adsorption material containing a 2:1 type layered clay mineral as represented by the following general formula:
[(E1^{m+}_{a/m}E2⁺_{b})(M1_{c}M2_{d})(Si₄₋ₑAlₑ)O₁₀(OH_{f}F_{2-f})]
where
m is a natural number of 2 to 4,
parameters a, b, c, d, e and f satisfy inequalities: 0.2≦a+b<0.75, 0.12≦a≦0.6, 0≦b, 0≦c≦3.0, 0≦d≦2, 2≦c+d ≦3, 0≦e<4, and 0≦f≦2,
E1 is at least one element selected from the group consisting of Mg, Al, Si, Sc, Ca, Sr, Ti, V, Cr, Mn, Fe, Co, Ni, Cu, Zn, Ga, Zr and Ba, said element turning into a polyvalent cation between layers,
E2 is at least one element selected from the group consisting of Na, Li and K, said element turning into a monovalent cation between layers,
M1 is at least one metal element selected from the group consisting of Mg, Fe, Mn, Ni, Zn and Li, and
M2 is at least one metal element selected from the group consisting of Al, Fe, Mn and Cr, wherein:
the M1 and M2 form an octahedral sheet.

2. The purine base adsorption material according to claim 1, wherein the 2:1 type layered clay mineral is at least one smectite selected from the group consisting of montmorillonite, beidellite, nontronite, saponite, hectorite and stevensite.

3. The purine base adsorption material according to claim 1 or 2, wherein the parameters a and b satisfy 0.2 ≦a+b≦0.6.

4. The purine base adsorption material according to any one of claims 1 to 3, wherein the E1 is at least one element selected from the group consisting of Mg, Al, Si, Sc, Ca, Ti, V, Cr, Mn, Fe, Ni, Cu, Ga and Zr.

5. The purine base adsorption material according to claim 3, wherein an occupied area per polyvalent cation E1^{m+} on a layer plane is in a range of no less than 0.8 nm²/cation to no greater than 9 nm²/cation.

6. The purine base adsorption material according to any one of claims 1 to 5, wherein the 2:1 type layered clay mineral has a basal plane spacing of no less than 1.2 nm to no greater than 1.9 nm as measured by X-ray diffraction in a state wetted with water.

7. The purine base adsorption material according to any one of claims 1 to 6, wherein the 2:1 type layered clay mineral has a primary particle diameter measured as a constant direction (Green) diameter in the ab-axis direction of a crystal observed under a microscope of no less than 10nm to no greater than 2µm.

8. The purine base adsorption material according to any one of claims 1 to 7, which further contains at least one additive selected from the group consisting of active carbon, acid clay, activated clay and zeolite.

9. A purine base adsorption filter comprising a purine base adsorption material carried on a fibrous material, wherein the purine base adsorption material is the purine base adsorption material according to any one of claims 1 to 8.

10. A purine base adsorption column filler including a purine base adsorption material, wherein:
the purine base adsorption material is the purine base adsorption material according to any one of claims 1 to 8, and
the purine base adsorption material is spherically granulated.

11. A purine base removal system, comprising a feeding means for feeding a fluid containing a purine base,
a removal means for removing the purine base from the fluid fed from the feeding means, and
a recovery means for recovering the fluid from which the purine base is removed by the removal means, wherein the removal means comprises the purine base adsorption filter according to claim 9, or the purine base adsorption column filler according to claim 10.

## Patentansprüche

1. Ein Purinbase-Adsorptionsmaterial, das ein geschichtetes Tonmineral vom Typ 2:1 enthält, wie durch die folgende allgemeine Formel dargestellt:
[(E1^{m+}_{a/m}E2⁺_{b})(M1_{c}M2_{d})(Si₄₋ₑAlₑ)O₁₀(OH_{f}F_{2-f})]
wobei
m eine natürliche Zahl von 2 bis 4 ist,
Parameter a, b, c, d, e und f Ungleichungen erfüllen:
0,2≤a+b<0,75, 0,12≤a≤0,6, 0≤b, 0≤c≤3,0, 0≤d≤2, 2≤c+d≤3, 0≤e<4 und 0≤f≤2,
E1 mindestens ein Element, ausgewählt aus der Gruppe bestehend aus Mg, Al, Si, Sc, Ca, Sr, Ti, V, Cr, Mn, Fe, Co, Ni, Cu, Zn, Ga, Zr und Ba, ist, wobei sich das Element in ein mehrwertiges Kation zwischen Schichten verwandelt,
E2 mindestens ein Element, ausgewählt aus der Gruppe bestehend aus Na, Li und K, ist, wobei sich das Element in ein einwertiges Kation zwischen Schichten verwandelt,
M1 mindestens ein Metallelement, ausgewählt aus der Gruppe bestehend aus Mg, Fe, Mn, Ni, Zn und Li, ist und
M2 mindestens ein Metallelement, ausgewählt aus der Gruppe bestehend aus Al, Fe, Mn und Cr, ist, wobei:
das M1 und das M2 eine oktaedrische Bahn bilden.

2. Das Purinbase-Adsorptionsmaterial nach Anspruch 1, wobei das geschichtete Tonmineral vom Typ 2:1 mindestens ein Smektit, ausgewählt aus der Gruppe bestehend aus Montmorillonit, Beidellit, Nontronit, Saponit, Hektorit und Stevensit, ist.

3. Das Purinbase-Adsorptionsmaterial nach Anspruch 1 oder 2, wobei die Parameter a und b 0,2≤a+b≤0,6 erfüllen.

4. Das Purinbase-Adsorptionsmaterial nach einem der Ansprüche 1 bis 3, wobei das E1 mindestens ein Element, ausgewählt aus der Gruppe bestehend aus Mg, Al, Si, Sc, Ca, Ti, V, Cr, Mn, Fe, Ni, Cu, Ga und Zr, ist.

5. Das Purinbase-Adsorptionsmaterial nach Anspruch 3, wobei ein belegter Bereich pro mehrwertigem Kation E1^{m+} auf einer Schichtebene in einem Bereich von nicht weniger als 0,8 nm²/Kation bis nicht mehr als 9 nm²/Kation liegt.

6. Das Purinbase-Adsorptionsmaterial nach einem der Ansprüche 1 bis 5, wobei das geschichtete Tonmineral vom Typ 2:1 einen Basalebenenabstand von nicht weniger als 1,2 nm bis nicht mehr als 1,9 nm aufweist, wie durch Röntgenbeugung in einem mit Wasser befeuchteten Zustand gemessen.

7. Das Purinbase-Adsorptionsmaterial nach einem der Ansprüche 1 bis 6, wobei das geschichtete Tonmineral vom Typ 2:1 einen Primärteilchendurchmesser, gemessen als Durchmesser bei konstanter Richtung (Green) in der ab-Achsrichtung eines unter einem Mikroskop betrachteten Kristalls, von nicht weniger als 10 nm und nicht mehr als 2 µm aufweist.

8. Das Purinbase-Adsorptionsmaterial nach einem der Ansprüche 1 bis 7, welches weiter mindestens ein Additiv, ausgewählt aus der Gruppe bestehend aus Aktivkohle, saurem Ton, Aktivton und Zeolith, enthält.

9. Ein Purinbase-Adsorptionsfilter, umfassend ein auf einem faserförmigen Material geträgertes Purinbase-Adsorptionsmaterial, wobei das Purinbase-Adsorptionsmaterial das Purinbase-Adsorptionsmaterial nach einem der Ansprüche 1 bis 8 ist.

10. Ein Purinbase-Adsorptionssäulenfüllstoff, der ein Purinbase-Adsorptionsmaterial beinhaltet, wobei:
das Purinbase-Adsorptionsmaterial das Purinbase-Adsorptionsmaterial nach einem der Ansprüche 1 bis 8 ist und
das Purinbase-Adsorptionsmaterial kugelförmig granuliert ist.

11. Ein Purinbase-Entfernungssystem, umfassend eine Zufuhrvorrichtung zur Zufuhr eines Fluids, das eine Purinbase enthält,
eine Entfernungsvorrichtung zur Entfernung der Purinbase aus dem Fluid, das aus der Zufuhrvorrichtung zugeführt wird, und
eine Gewinnungsvorrichtung zur Gewinnung des Fluids, aus der die Purinbase mit Hilfe der Entfernungsvorrichtung entfernt wurde, wobei die Entfernungsvorrichtung den Purinbase-Adsorptionsfilter nach Anspruch 9 oder den Purinbase-Adsorptionssäulenfüllstoff nach Anspruch 10 umfasst.

## Revendications

1. Matériau d'adsorption à base de purine contenant un minéral d'argile en couches de type 2:1 comme représenté par la formule générale suivante :
[(E1^{m+}_{a/m}E2⁺_{b})(M1_{c}M2_{d})(Si₄₋ₑAlₑ)O₁₀(OH_{f}F_{2-f})]
où
m est un nombre entier de 2 à 4,
les paramètres a, b, c, d, e et f satisfont les inégalités : 0,2≤a+b<0,75, 0,12≤a≤0,6, 0≤b, 0≤c≤3,0, 0≤d≤2, 2≤c+d≤3, 0≤e≤4, et 0≤f≤2,
E1 est au moins un élément choisi dans le groupe consistant en Mg, Al, Si, Sc, Ca, Sr, Ti, V, Cr, Mn, Fe, Co, Ni, Cu, Zn, Ga, Zr et Ba, ledit élément se convertissant en un cation polyvalent entre les couches,
E2 est au moins un élément choisi dans le groupe consistant en Na, Li et K, ledit élément se convertissant en un cation monovalent entre les couches,
M1 est au moins un élément de métal choisi dans le groupe consistant en Mg, Fe, Mn, Ni, Zn et Li, et
M2 est au moins un élément de métal choisi dans le groupe consistant en Al, Fe, Mn et Cr, dans lequel :
les M1 et M2 forment une feuille octaédrique.

2. Matériau d'adsorption à base de purine selon la revendication 1, dans lequel le minéral d'argile en couches de type 2:1 est au moins une smectite choisie dans le groupe consistant en montmorillonite, beidellite, nontronite, saponite, hectorite et stevensite.

3. Matériau d'adsorption à base de purine selon la revendication 1 ou 2, dans lequel les paramètres a et b satisfont 0,2≤a+b≤0,6.

4. Matériau d'adsorption à base de purine selon l'une quelconque des revendications 1 à 3, dans lequel le E1 est au moins un élément choisi dans le groupe consistant en Mg, Al, Si, Sc, Ca, Ti, V, Cr, Mn, Fe, Ni, Cu, Ga et Zr.

5. Matériau d'adsorption à base de purine selon la revendication 3, dans lequel une surface occupée par cation polyvalent E1^{m+} sur un plan de couche se trouve dans un intervalle d'au moins 0,8 nm²/cation à au plus 9 nm²/cation.

6. Matériau d'adsorption à base de purine selon l'une quelconque des revendications 1 à 5, dans lequel le minéral d'argile en couches de type 2:1 présente un espacement de plan de base d'au moins 1,2 nm à au plus 1,9 nm comme mesuré par diffraction de rayons X dans un état mouillé avec de l'eau.

7. Matériau d'adsorption à base de purine selon l'une quelconque des revendications 1 à 6, dans lequel le minéral d'argile en couches de type 2:1 présente un diamètre de particule primaire mesuré comme un diamètre de direction constante (Green) dans la direction d'axe-ab d'un cristal observé sous un microscope d'au moins 10 nm à au plus 2 µm.

8. Matériau d'adsorption à base de purine selon l'une quelconque des revendications 1 à 7, qui contient de plus au moins un additif choisi dans le groupe consistant en charbon actif, argile acide, argile activée et zéolite.

9. Filtre d'adsorption à base de purine comprenant un matériau d'adsorption à base de purine supporté sur un matériau fibreux, dans lequel le matériau d'adsorption à base de purine est le matériau d'adsorption à base de purine selon l'une quelconque des revendications 1 à 8.

10. Charge de colonne d'adsorption à base de purine incluant un matériau d'adsorption à base de purine, dans laquelle :
le matériau d'adsorption à base de purine est le matériau d'adsorption à base de purine selon l'une quelconque des revendications 1 à 8, et
le matériau d'adsorption à base de purine est sphériquement granulé.

11. Système d'élimination à base de purine, comprenant un moyen d'alimentation pour introduire un fluide contenant une base de purine,
un moyen d'élimination pour éliminer la base de purine du fluide introduit par le moyen d'alimentation, et
un moyen de récupération pour récupérer le fluide à partir duquel la base de purine est éliminée par le moyen d'élimination, dans lequel le moyen d'élimination comprend le filtre d'adsorption à base de purine selon la revendication 9, ou la charge de colonne d'adsorption à base de purine selon la revendication 10.
